# EUROPEAN PATENT SPECIFICATION

(11) **EP 3 983 340 B1**
(45) Date of publication and mention of the grant of the patent: **02.08.2023**
(21) Application number: 20730679.6
(22) Date of filing: 11.06.2020
(51) Int. Cl.: C02F 1/42, C02F 1/46, C07C 51/02, C07C 51/48, C07C 51/487, C07C 407/00, C07C 53/122, C07C 53/124, C07C 53/126, C07C 53/128, C07C 53/132, C07C 53/134, C02F 1/26, C02F 1/469, C02F 101/34

(54) **METHOD FOR ISOLATING CARBOXYLIC ACID FROM AN AQUEOUS SIDE STREAM**
VERFAHREN ZUR ISOLIERUNG VON CARBONSÄURE AUS EINEM WÄSSRIGEN SEITENSTROM
PROCÉDÉ D'ISOLATION D'ACIDE CARBOXYLIQUE À PARTIR D'UN FLUX LATÉRAL AQUEUX

(30) Priority: 12.06.2019 EP 19179625
(43) Date of publication of application: 20.04.2022
(73) Proprietor: Nouryon Chemicals International B.V., 6824 BM Arnhem (NL)
(72) Inventor: TAMMER, Martinus Catharinus, 7418 AJ Deventer (NL); BART, Jacob, 7418 AJ Deventer (NL); LAMMERS, Hans, 7418 AJ Deventer (NL)
(74) Representative: LKGlobal UK Ltd.
(86) International application number: PCT/EP2020/066232
(87) International publication number: WO 2020/249692

(56) References cited:
- EP-A1- 2 666 763
- EP-A2- 0 323 663
- EP-B1- 0 616 505
- EP-B1- 0 682 695
- EP-B1- 3 047 845
- AU-A- 5 310 473
- CA-A- 488 970
- CA-A1- 2 167 279
- CN-A- 1 061 777
- CN-A- 1 151 421
- CN-A- 1 343 247
- CN-A- 1 368 981
- CN-A- 1 427 771
- CN-A- 1 463 282
- CN-A- 1 519 649
- CN-A- 1 537 131
- CN-A- 1 575 204
- CN-A- 1 660 498
- CN-A- 1 720 266
- CN-A- 1 745 069
- CN-A- 1 847 289
- CN-A- 1 860 197
- CN-A- 1 933 808
- CN-A- 1 938 291
- CN-A- 1 942 471
- CN-A- 1 946 723
- CN-A- 101 107 254
- CN-A- 101 133 091
- CN-A- 101 249 046
- CN-A- 101 249 047
- CN-A- 101 263 130
- CN-A- 101 522 177
- CN-A- 101 522 570
- CN-A- 102 076 317
- CN-A- 102 076 318
- CN-A- 102 076 319
- CN-A- 102 088 956
- CN-A- 102 093 909
- CN-A- 102 131 773
- CN-A- 102 574 933
- CN-A- 102 666 614
- CN-A- 102 844 054
- CN-A- 102 858 940
- CN-A- 103 228 272
- CN-A- 103 622 842
- CN-A- 104 394 835
- CN-A- 105 765 672
- CN-A- 105 793 344
- CN-A- 105 813 617
- CN-A- 105 979 969
- CN-A- 106 029 052
- CN-A- 108 423 908
- CN-B- 1 911 971
- CN-B- 1 986 635
- CN-B- 1 997 340
- CN-B- 101 072 779
- CN-B- 102 627 778
- CN-B- 102 858 944
- CN-B- 103 044 645
- CN-B- 103 649 278

## Description

This invention relates to a method for isolating carboxylic acid from an aqueous side stream of an organic peroxide production process.

Diacyl peroxides and peroxyesters can be prepared by reacting an anhydride or acid chloride with alkaline solutions of hydro(gen)peroxide, as illustrated by the following equations:

2 R-C(=O)-O-C(=O)-R + Na₂O₂ → R-C(=O)-O-O-C(=O)-R + 2 NaOC(=O)R

R-C(=O)-O-C(=O)-R + ROOH + NaOH → R-C(=O)-O-O-R + NaOC(=O)R

2 R-C(=O)CI + Na₂O₂ → R-C(=O)-O-O-C(=O)-R + 2 NaCl

R-C(=O)CI + ROOH + NaOH → R-C(=O)-O-O-R + NaCl

In this reaction scheme, Na₂O₂ does not refer to a discrete product Na₂O₂, but to an equilibrium comprising H₂O₂ and NaOOH.

Acid chlorides are relatively expensive and generate chloride-containing water layers, which lead to waste waters with high salt concentration.

Anhydrides, on the other hand, are even more expensive than acid chlorides and the side stream of the process starting with anhydride contains a high organic load - i.e. a high Chemical Oxygen Demand (COD) value - due to the formed carboxylic acid salt, and is therefore economically and environmentally unattractive.

That would change if the carboxylic acid could be isolated from the aqueous side stream and be re-used; either in a peroxide production process, in another chemical process (e.g. the production of esters), or in any other application, e.g. as animal feed ingredient.

CN108423908 discloses a process to isolate 4-methylbenzoic acid from a bis(4-methylbenoyl)peroxide production process waste stream by precipitation. However, this process only works for acids with low solubility in water. In addition, the precipitate can cause smearing of the equipment used.

For carboxylic acids that are water-soluble or do not sufficiently precipitate or otherwise separate from the aqueous side stream, isolation is not easy or straightforward.

It is therefore an object of the present invention to provide a method for isolating such carboxylic acids from an aqueous side stream of an organic peroxide production process and make it suitable for re-use.

This object is achieved by a process comprising the following steps:
a) providing an aqueous side stream of an organic peroxide production process, said stream comprising at least 1 wt% of a metal carboxylate, said metal carboxylate being dissolved or homogeneously admixed within said stream,
b) protonating the carboxylate towards carboxylic acid inside the aqueous side stream, thereby forming a biphasic mixture of two liquid phases,
c) separating the biphasic mixture in (i) an aqueous liquid phase comprising water and a minor amount of carboxylic acid and (ii) an organic liquid phase comprising carboxylic acid and a minor amount of water,
d) optionally, separating, preferably distilling, the carboxylic acid from said organic liquid phase,
wherein residual peroxides present in the aqueous side stream are removed by (i) extraction before or after step b) and/or (ii) the addition of a reducing agent, heat or irradiation to said stream, to the biphasic mixture, and/or to the organic liquid phase.

The aqueous side stream is preferably obtained from the production of diacyl peroxides and/or peroxyesters. The organic peroxide production process leading to said aqueous side stream may involve the use of an acid chloride or an anhydride as reactant, preferably an anhydride.

It may be noted that EP 2 666 763 discloses a process to recover carboxylic acid from a magnesium carboxylate mixture by employing an acidic ion exchanger that replaces the magnesium ions in the carboxylate with a proton and in this way provides carboxylic acid. This document however does not relate to recovery from peroxide process streams and also does not involve any further biphasic liquid-liquid separation to recover the carboxylic acid.

Diacyl peroxides can be symmetrical or asymmetrical.

Examples of suitable symmetrical diacyl peroxides are di-2-methylbutyryl peroxide, di-isovaleryl peroxide, di-n-valeryl peroxide, di-n-caproyl peroxide, di-isobutyryl peroxide, and di-n-butanoyl peroxide.

Examples of suitable asymmetrical diacyl peroxides are acetyl isobutanoyl peroxide, acetyl 3-methylbutanoyl peroxide, acetyl lauroyl peroxide acetyl isononanoyl peroxide, acetyl heptanoyl peroxide, acetyl cyclohexylcarboxylic peroxide, acetyl 2-propylheptanoyl peroxide, and acetyl 2-ethylhexanoyl peroxide.

Examples of suitable peroxyesters are tert-butylperoxy 2-ethylhexanoate, tert-amylperoxy 2-ethylhexanoate, tert-hexylperoxy 2-ethylhexanoate, 1,1,3,3-tetramethyl butyl-1-peroxy 2-ethylhexanoate, 1,1 ,3,3-tetramethylbutyl 1-peroxyneodecanoate, tert- butylperoxy neodecanoate, tert-amylperoxy neodecanoate, tert-hexylperoxy neodecanoate, 1,1 ,3,3-tetramethylbutyl 1-peroxyneoheptanoate, tert-butylperoxy neoheptanoate, tert-amylperoxy neoheptanoate, tert-hexylperoxy neoheptanoate, 1,1 ,3,3-tetramethylbutyl 1-peroxyneononanoate, tert-butylperoxy neononanoate, tert-amylperoxy neononanoate, tert-hexylperoxy neononanoate, tert-butylperoxy pivalate, tert-amylperoxy pivalate, tert-hexylperoxy pivalate, 1,1,3,3-tetramethyl butyl-1-peroxy pivalate, tert-butylperoxy 3,3,5-trimethylhexanoate, tert-amylperoxy 3,3,5-trimethylhexanoate, tert-hexylperoxy 3,3,5-trimethylhexanoate, 1,1,3,3-tetramethyl butyl-1-peroxy 3,3,5-trimethylhexanoate, tert-butylperoxy isobutyrate, tert-amylperoxy isobutyrate, tert-hexylperoxy isobutyrate, 1,1,3,3-tetramethyl butyl-1-peroxy isobutyrate, tert-butylperoxy n-butyrate, tert-amylperoxy n-butyrate, tert-hexylperoxy n-butyrate, tert-butylperoxy isovalerate, tert-amylperoxy isovalerate, , tert-hexylperoxy isovalerate, 1,1,3,3-tetramethyl butyl-1-peroxy isovalerate, tert-butylperoxy n-valerate, tert-amylperoxy n-valerate, tert-hexylperoxy n-valerate, 1,1,3,3-tetramethyl butyl-1-peroxy n-butyrate, 1,1,3,3-tetramethyl butyl 1-peroxy m-chlorobenzoate, tert-butylperoxy m-chlorobenzoate, tert-amylperoxy m-chlorobenzoate, tert-hexylperoxy m-chlorobenzoate, 1,1,3,3-tetramethyl butyl 1-peroxy o-methylbenzoate, tert-butylperoxy o-methylbenzoate, tert-amylperoxy o-methylbenzoate, tert-hexylperoxy o-methylbenzoate, 1,1,3,3-tetramethyl butyl 1-butylperoxy phenylacetate, tert-butylperoxy phenylacetate, tert-amylperoxy phenylacetate, tert-hexylperoxy phenylacetate, tert-butylperoxy 2-chloroacetate, tert-butylperoxy cyclododecanoate, tert-butylperoxy n-butyrate, tert-butylperoxy 2-methylbutyrate, tert-amylperoxy 2-methylburyrate, 1,1-dimethyl-3-hydroxy butyl-1-peroxy neodecanoate, 1,1-dimethyl-3-hydroxy butyl-1-peroxy pivalate, 1,1-dimethyl-3-hydroxy butyl-1-peroxy 2-ethylhexanoate, 1,1-dimethyl-3-hydroxy butyl-1-peroxy 3,3,5-trimethylhexanoate, and 1,1-dimethyl-3-hydroxy butyl-1-peroxy isobutyrate.

Preferred peroxyesters include tert-butylperoxy isobutyrate, tert-amylperoxy isobutyrate, 1,1,3,3-tetramethyl butyl-1-peroxy isobutyrate, tert-butylperoxy n-butyrate, tert-amylperoxy n-butyrate, 1,1,3,3-tetramethyl butyl-1-peroxy n-butyrate, tert-butylperoxy isovalerate, tert-amylperoxy isovalerate, tert-butylperoxy 2-methylbutyrate, tert-amylperoxy 2-methylburyrate, 1,1,3,3-tetramethyl butyl-1-peroxy isovalerate, tert-butylperoxy n-valerate, tert-amylperoxy n-valerate, and 1,1,3,3-tetramethyl butyl-1-peroxy n-valerate.

The aqueous side stream of an organic peroxide production process comprises at least 1 wt%, preferably at least 3 wt%, more preferably at least 5 wt%, more preferably at least 10 wt%, even more preferably at least 20 wt%, and most preferably at least 25 wt% of a metal carboxylate dissolved or homogeneously admixed therein. The metal carboxylate concentration is preferably not more than 50 wt%, more preferably not more than 40 wt%, and most preferably not more than 35 wt%.

The metal carboxylate is dissolved or homogeneously admixed with said stream, meaning that the stream consists of a single phase and is not, e.g., a suspension containing metal carboxylate particles. From such a suspension, the carboxylic acid could be easily separated by, e.g., filtration of the metal carboxylate. From the aqueous stream of the present invention, however, such easy separation is not possible and more steps are required to isolate the carboxylic acid.

Apart from water and the metal carboxylate, the aqueous side stream will contain some peroxide residues, such as organic hydroperoxide, hydrogen peroxide, peroxyacid, diacyl peroxide, and/or peroxyester. The peroxide content of the aqueous side stream will generally be in the range 0.01-3 wt%. The side stream may further contain some residual peroxide decomposition products.

In order to successfully isolate, purify, and re-use the carboxylic acid, any residual peroxides have to be removed from the aqueous side stream. This is done by extraction and/or the addition of a reducing agent. In addition, heating of the side stream may be desired.

Examples of suitable reducing agents are sodium sulfite, sodium (poly)sulfide (Na₂Sₓ), sodium thiosulfate, and sodium metabisulfite.

Reducing agent is added to the aqueous side stream, to the biphasic mixture, and/or to the organic liquid phase. In a preferred embodiment, reducing agent is added to the aqueous side stream, either during step b) or, more preferably, before step b).

The reducing agent will destroy hydrogen peroxide, organic hydroperoxides, and peroxy acids. In order to destroy any other peroxidic species, it may be desired to increase the temperature of the aqueous side stream with 10-80°C, preferably 10-50°C, and most preferably 10-30°C. This temperature increase can be performed before step b) or during step b). If performed during step b), any heat that is liberated by the protonation (e.g. acidification) may be used to achieve this temperature increase.

It should be noted that the temperature of the aqueous side stream before heating or protonation is generally in the range 0-20°C, preferably 0-10°C, as peroxide production processes are often performed at low temperatures.

Extraction can be performed before or after step b), and is preferably performed before step b). Extraction can be performed with organic solvents, anhydrides, and mixtures of anhydride and solvent.

Examples of suitable solvents for the extraction are alkanes (e.g. isododecane, Spiridane^{®} and Isopar^{®} mineral oils), chloroalkanes, esters (e.g. ethyl acetate, methyl acetate, dimethylphthalate, ethylene glycol dibenzoate, cumene, dibutyl maleate, di-isononyl-1,2-cyclohexaendicarboxylate (DINCH), dioctyl terephthalate, or 2,2,4-trimethylpentanediol diisobutyrate (TXIB)), ethers, amides, and ketones.

Examples of suitable anhydrides are anhydrides that were or can be used in the organic peroxide production process and include symmetrical and asymmetrical anhydrides. Examples of symmetrical anhydrides are n-butyric anhydride, isobutyric anhydride, pivalic anhydride, valeric anhydride, isovaleric anhydride, 2-methylbutyric anhydride, 2-methylpentanoic anhydride, 2-methylhexanoic anhydride, 2-methylheptanoic anhydride 2-ethylbutyric anhydride, caproic anhydride, caprylic anhydride, isocaproic anhydride, n-heptanoic anhydride, nonanoic anhydride, isononanoic anhydride, 3,5,5-trimethylhexanoic anhydride, 2-propylheptanoic anhydride, decanoic anhydride, neodecanoic anhydride, undecanoic anhydride, neoheptanoic anhydride, lauric anhydride, tridecanoic anhydride, 2-ethylhexanoic anhydride, myristic anhydride, palmitic anhydride, stearic anhydride, phenylacetic anhydride, cyclohexanecarboxylic anhydride, 3-methyl-cyclopentanecarboxylic anhydride, and mixtures of two or more of the above-mentioned anhydrides.

Examples of suitable mixtures of symmetrical anhydrides are the mixture of isobutyric anhydride and 2-methylbutyric anhydride, the mixture of isobutyric anhydride and 2-methylpentanoic anhydride, the mixture of 2-methylbutyric anhydride and isovaleric anhydride, and the mixture of 2-methylbutyric anhydride and valeric anhydride.

Asymmetrical anhydrides are usually available as a mixture of the asymmetrical and symmetrical anhydrides. This is because asymmetrical anhydrides are usually obtained by reacting a mixture of acids with, e.g., acetic anhydride. This leads to a mixture of anhydrides, including an asymmetrical and at least one symmetrical anhydride. Such mixtures of anhydrides can be used for the extraction. Examples of suitable asymmetrical anhydrides are isobutyric 2-methylbutyric anhydride, which is preferably present as admixture with isobutyric anhydride and 2-methylbutyric anhydride; isobutyric acetic anhydride, which is preferably present as admixture with isobutyric anhydride and acetic anhydride, 2-methylbutyric valeric anhydride which is preferably present as admixture with 2-methylbutyric anhydride and valeric anhydride; and butyric valeric anhydride, which is preferably present as admixture with butyric anhydride and valeric anhydride.

More preferred anhydrides are isobutyric anhydride, 2-methylbutyric anhydride, 2-methylhexanoic anhydride, 2-propylheptanoic anhydride, n-nonanoic anhydride, isononanoic anhydride, cyclohexanecarboxylic anhydride, 2-ethylhexanoic anhydride, caprylic anhydride, n-valeric anhydride, isovaleric anhydride, caproic anhydride, and lauric anhydride. Most preferred are isononanoic anhydride and isobutyric anhydride.

In step b), the carboxylic acid is liberated by protonation. Protonation leads to a biphasic mixture of two liquid phases. In other words: it does not lead to precipitation of the carboxylic acid which could then be easily separated from the mixture by, e.g., filtration. Instead, from the mixture of the present invention, such easy separation is not possible, and more steps are required to isolate the carboxylic acid.

In one embodiment, protonation is achieved by acidification of the aqueous side stream.

Preferred acids for acidifying and protonating the carboxylic acid are acids with a pKₐ below 5, such as H₂SO₄, HCl, NaHSO₄, KHSO₄, formic acid, acetic acid, and combinations thereof. More preferably, an acid with a pKₐ below 3 is used; most preferably H₂SO₄ is used. If H₂SO₄ is used, it is preferably added as a 90-96 wt% solution.

Acidification is preferably performed to a pH below 6, more preferably below 4.5, and most preferably below 3. The resulting pH is preferably not lower than 1. Depending on the acid used, the temperature of the stream may increase during this step, up to about 80°C.

Acidification leads to the formation of a biphasic mixture comprising (i) an aqueous layer comprising water and a minor amount of carboxylic acid and (ii) an organic liquid phase comprising carboxylic acid and a minor amount of water. The salt that results from the acidification - e.g. Na₂SO₄, K₂SO₄, NaHSO₄, KHSO₄, NaCl, Na formate, or Na acetate, depending on the acid used for acidification and the base used during the organic peroxide production - will be mainly present in the aqueous liquid phase, although a minor amount may also be present in the organic liquid phase.

In this document a minor amount is defined as 0 to 2 wt% based on total weight, preferably less than 1 wt%, more preferably less than 0.5 wt%, more preferably less than 0.1 wt%, more preferably less than 0.01 wt% and most preferably less than 0.001 wt%.

In another embodiment, protonation is achieved by electrochemical membrane separation. Examples of electrochemical membrane separation techniques are membrane electrolysis and bipolar membrane electrodialysis (BPM). BPM is the preferred electrochemical membrane separation method.

Electrochemical membrane separation leads to splitting of the metal carboxylate in carboxylic acid and metal hydroxide (e.g. NaOH or KOH) and separation of both species. It thus leads to (i) a carboxylic acid-containing mixture and (ii) a NaOH or KOH solution, separated by a membrane.

The NaOH or KOH solution can be re-used in the production of organic peroxides or any of the steps of the process of the present invention where a base is required or desired.

Depending on the temperature, the salt concentration, and the solubility of the carboxylic acid in water, the carboxylic acid-containing mixture can be a biphasic mixture of two liquid phases or a homogeneous mixture. If a homogeneous mixture is formed under the electrochemical membrane separation conditions (generally 40-50°C), cooling of the mixture to temperatures below about 30°C and/or the addition of salt will ensure that a biphasic mixture will be formed. The organic liquid layer of this biphasic carboxylic acid-containing mixture can then be separated from the aqueous layer of said biphasic mixture in step c).

Optionally, a solvent is added to the biphasic mixture.

Examples of suitable solvents are (mixtures of) alkanes like isododecane, Spirdane^{®}, Isopar^{®}, octane, decane, toluene, o-, m-, p- xylene, esters like dimethylphthalate, long chain acetates, butyl acetate, ethyl butyrate, cumene, trimethyl pentanyl diisobutyrate (TXIB), adipates, sebacates, maleates, trimellitates, azelates, benzoates, citrates, and terephthalates, ethers like methyl tert-butyl ether (MTBE), and carbonates like diethyl carbonate.

Alkanes and mixtures of alkanes are the preferred solvents. Isododecane is the most preferred solvent.

The addition of solvent is particularly desired if the carboxylic acid is to be re-used in a process in which said solvent is desirably present, so that the solvent does not need to be removed from the carboxylic acid before such re-use. Examples of such processes are organic peroxide production processes, in which safety considerations often require the presence of solvent.

In step c), the liquid phases are separated.

Separation can be performed by gravity, using conventional separation equipment, such as a liquid/liquid separator, a centrifuge, a (pulsed and or packed) counter current column, (a combination of) mixer settlers, or a continues (plate) separator.

In some embodiments, the separation can be facilitated by salting out the organic liquid phase with a concentrated salt solution, e.g. a 20-30 wt% NaCl, NaHSO₄, KHSO₄, Na₂SO₄, or K₂SO₄ solution. The salt reduces the solubility of the carboxylic acid in the aqueous liquid phase. This extraction can be performed in any suitable device, such as a reactor, centrifuge, or mixer-settler.

The preferred separation method in step c) is gravity separation, instead of extraction.

Irrespective of how the phases are separated in step c), a separation or distillation step d) is preferred to further purify the carboxylic acid. Distillation is especially preferred for the purification of carboxylic acids with less than five carbon atoms and for organic liquid phases with a water content of 5 wt% or more. For lower water contents, drying with a molecular sieve or drying salt can be performed to remove water.

The distillation may serve to evaporate volatile impurities, including water, from the carboxylic acid and/or to distill the carboxylic acid from any impurities with a boiling point higher than that of the carboxylic acid.

The term "distillation" in this specification includes any form of removal of components by vaporization. Hence, it also includes stripping and similar techniques.

Between separation step c) and separation or distillation step d), it may be desired to remove any salts - that resulted from the acidification - from the organic liquid phase, in order to prevent settling of solids in the distillation column. Removal of salt can be done by washing with water, cooling (e.g. freezing), and separating off the resulting water layer. Cooling is preferably performed to <20°C, more preferably <10°C, and most preferably <5°C and will force salts into the water layer.

The isolated water layer can be recycled to the protonation step.

The water content of the obtained carboxylic acid is preferably below 2 wt%, more preferably below 1 wt%, even more preferably below 0.5 wt%, and most below 0.1 wt%. This is especially preferred in case the carboxylic acid will be re-used in a peroxide production process. A further distillation of the carboxylic acid may be required to reach this water content.

The aqueous liquid phase formed as a result of protonation step b) may contain some residual carboxylic acid. This holds in particular for lower molecular weight acids, like butyric, isobutyric, pentanoic, and methyl- or ethyl-branched pentanoic acids. This residual acid can be recovered by adsorption, (azeotropic) distillation, or extraction, preferably distillation. Optionally, a salt (e.g. sodium sulfate) can be added to the recovered carboxylic acid as an aqueous liquid distillate, in order to lower the solubility of the carboxylic acid. In order to further optimize the carboxylic acid yield, the recovered residual carboxylic acid distillate can be recycled within the process by adding it to the above-mentioned aqueous side stream, after protonation step b) and before separation step c).

Preferred carboxylic acids to be obtained by the process of the present invention include isobutyric acid, n-butyric acid, propionic acid, pivalic acid, neodecanoic acid, neoheptanoic acid, isononanoic acid, 2-methylbutyric acid, cyclohexylcarboxylic acid, lauric acid, isovaleric acid, n-valeric acid, n-hexanoic acid, 2-ethylhexanoic acid, heptanoic acid, 2-propylheptanoic acid, octanoic acid, nonanoic acid, decanoic acid, and lauric acid. More preferred carboxylic acids are isobutyric acid, n-butyric acid, n-heptanoic acid, n-octanoic acid, pivalic acid, isononanoic acid, 2-methylbutyric acid, cyclohexylcarboxylic acid, isovaleric acid, and n-valeric acid.

The carboxylic acid obtained from the process of the present invention can be recycled to the organic peroxide production process from which it originated, it can be used in the production of another organic peroxide, and it can be used to make esters (for instance ethyl esters) that find use as, e.g., solvent or fragrance, or in agricultural applications.

The carboxylic acid - or a salt thereof - can also be used in animal feed. For instance, butyric acid salts are known to improve gastrointestinal health in poultry and prevent microbial infections and ailments in poultry, pigs, fishes, and ruminants.

### EXAMPLE

An aqueous side stream of a di-isobutyryl peroxide process containing 23 wt% sodium isobutyrate, 300 ppm di-isobutyryl peroxide and 0.1 wt% perisobutyric acid, having a temperature of 0°C and a pH of about 10, was treated as follows: A constant flow of said stream was passed through a column with four stirred sections and kept at a temperature of 20-25°C. The residence time was about 5 minutes per section. A 30 wt% Na₂SO₃ solution was added to said column, thereby reducing the perisobutyric acid in said stream and producing a stream with <50 ppm of residual peroxide.

The resulting stream was collected in a container.

From the container, 8.2 kg of said stream was charged to a 10 l glass reactor, equipped with a cooling mantle, a pitch blade impeller, and a thermometer. To the stirred contents, 810.4 g of H₂SO₄-96 wt% was added in 2 minutes in order to reduce the pH to 2.3. A temperature increase to 42°C was noticed. After 5 minutes of stirring, the layers were allowed to separate by gravity. The two phases were separated, thereby obtaining 7.6 kg of aqueous liquid phase and 1.4 kg of organic liquid phase.

Cooling of the organic phase to 2°C resulted in a separation of 40 g of additional aqueous phase, which was then combined with the 7.6 kg aqueous liquid phase.

The organic liquid phase, mainly comprising wet isobutyric acid, was fed to a continuous distillation column. The bottom stream contained >99 wt% isobutyric acid with a water content of 200 ppm.

The aqueous liquid phase was charged to a 10 l glass reactor and an aqueous solution of isobutyric acid in water was distilled off at 55°C and <160 mbar. The residue was an aqueous Na₂SO₄ solution.

In order to further optimize the isobutyric acid yield, this aqueous isobutyric acid solution was recycled within the process by adding it to the above-mentioned aqueous side stream, after the acidification with H₂SO₄ and before gravity separation of the resulting layers.

## Claims

1. Method for isolating carboxylic acid from an aqueous side stream of an organic peroxide production process, said method comprising the steps of:
a) providing an aqueous side stream of an organic peroxide production process, said stream comprising at least 1 wt% of a metal carboxylate, said metal carboxylate being dissolved or homogeneously admixed within said stream,
b) protonating the carboxylate towards carboxylic acid inside the aqueous side stream, thereby forming a biphasic mixture of two liquid phases,
c) separating the biphasic mixture in (i) an aqueous liquid phase comprising water and a minor amount of carboxylic acid and (ii) an organic liquid phase comprising carboxylic acid and a minor amount of water,
d) optionally, separating, preferably distilling, the carboxylic acid from said organic liquid phase,
wherein residual peroxides present in the aqueous side stream are removed by (i) extraction before or after step b) and/or (ii) the addition of a reducing agent, heat or irradiation to said stream, to the biphasic mixture, and/or to the organic liquid phase.

2. Method according to claim 1 wherein the aqueous side stream results from a diacyl peroxide or peroxyester production process.

3. Method according to claim 1 or 2 wherein the carboxylic acid is selected from the group consisting of isobutyric acid, n-butyric acid, propionic acid, pivalic acid, neodecanoic acid, neoheptanoic acid, isononanoic acid, 2-methylbutyric acid, cyclohexylcarboxylic acid, lauric acid, isovaleric acid, n-valeric acid, n-hexanoic acid, 2-ethylhexanoic acid, heptanoic acid, octanoic acid, nonanoic acid, decanoic acid, and lauric acid.

4. Method according to claim 3 wherein the carboxylic acid is selected from the group consisting of isobutyric acid, n-butyric acid, n-heptanoic acid, n-octanoic acid, pivalic acid, isononanoic acid, 2-methylbutyric acid, cyclohexylcarboxylic acid, isovaleric acid, and n-valeric acid.

5. Method according to any one of the preceding claims wherein the aqueous side stream in step a) comprises at least 3 wt%, preferably at least 5 wt%, more preferably at least 10 wt%, even more preferably at least 20 wt%, and most preferably at least 25 wt% of a metal carboxylate.

6. Method according to any one of the preceding claims wherein the protonation of the carboxylate towards carboxylic acid in step b) is performed by acidification of the aqueous side stream.

7. Method according to any one of claims 1-5 wherein the protonation of the carboxylate towards carboxylic acid in step b) is performed by electrochemical membrane separation, preferably bipolar membrane electrodialysis (BPM), of the aqueous side stream.

8. Method according to any one of the preceding claims wherein the peroxide present in the aqueous side stream is destroyed by the addition of a reducing agent to the aqueous side stream, either before or during step b), preferably before step b).

9. Method according claim 8 wherein the reducing agent is selected from the group consisting of sodium sulfite, sodium (poly)sulfide (Na₂Sₓ), sodium thiosulfate, and sodium metabisulfite.

10. Method according to any one of claims 1-9 wherein the phases are separated in step c) by gravity.

11. Method according to any one of claims 1-9 wherein the phases are separated in step c) by extraction with an organic solvent, preferably an alkane of mixture of alkanes, most preferably isododecane.

12. Method according to any one of claims 1-9 wherein the phases are separated in step c) by extraction with a salt solution, preferably a 20-30 wt% NaCl solution.

13. Method according to any one of the preceding claims comprising an additional step e) in which carboxylic acid is isolated from the aqueous liquid phase by distillation.

14. Method according to claim 13, further comprising recycling at least part of the carboxylic acid isolated in step e) to the biphasic mixture of step b).

15. Method as claimed in any preceding claim, further comprising recycling at least part of the carboxylic acid isolated in step c) or step d) to an organic peroxide production process, using the carboxylic acid isolated in step c) or step d) to make esters, or using the carboxylic acid isolated in step c) or step d) in animal feed.

## Patentansprüche

1. Verfahren zur Isolierung von Carbonsäure von einem wässrigen Seitenstrom eines Herstellungsverfahrens für organisches Peroxid, wobei das Verfahren die Schritte umfasst des
a) Bereitstellens eines wässrigen Seitenstroms eines Herstellungsverfahren für organisches Peroxid, wobei der Strom mindestens 1 Gew.-% eines Metallcarboxylats umfasst, wobei das Metallcarboxylat innerhalb des Stroms gelöst oder homogen beigemischt wird,
b) Protonierens des Carboxylats in Richtung auf die Carbonsäure innerhalb des wässrigen Seitenstroms, wodurch eine zweiphasige Mischung von zwei flüssigen Phasen gebildet wird,
c) Trennens der zweiphasigen Mischung in (i) eine wässrige flüssige Phase, die Wasser und eine geringe Menge Carbonsäure umfasst, und (ii) eine organische flüssige Phase, die Carbonsäure und eine geringe Menge Wasser umfasst,
d) wahlweise Trennens, bevorzugt Destillierens, der Carbonsäure von der organischen flüssigen Phase,
wobei die restlichen Peroxide, die in dem wässrigen Seitenstrom vorliegen, durch (i) Extraktion vor oder nach Schritt b) und/oder (ii) die Zugabe eines Reduktionsmittels, Wärme oder Bestrahlung zu dem Strom, zu der zweiphasigen Mischung und/oder zu der organischen flüssigen Phase entfernt werden.

2. Verfahren nach Anspruch 1, wobei der wässrige Seitenstrom aus einem Herstellungsverfahren für Diacylperoxid oder Peroxyester resultiert.

3. Verfahren nach Anspruch 1 oder 2, wobei die Carbonsäure aus der Gruppe ausgewählt wird bestehend aus Isobuttersäure, n-Buttersäure, Propionsäure, Pivalinsäure, Neodecansäure, Neoheptansäure,lsononansäure, 2-Methylbuttersäure,Cyclohexylcarbonsäure,Laurinsäure, Isovaleriansäure, n-Valeriansäure,n-Hexansäure,2-Ethylhexansäure, Heptansäure, Octansäure, Nonansäure und Decansäure und Laurinsäure.

4. Verfahren nach Anspruch 3, wobei die Carbonsäure aus der Gruppe ausgewählt wird bestehend aus Isobuttersäure, n-Buttersäure, n-Heptansäure, n-Octansäure, Pivalinsäure, Isononansäure, 2-Methylbuttersäure, Cyclohexylcarbonsäure, Isovaleriansäure und n-Valeriansäure.

5. Verfahren nach einem der vorhergehenden Ansprüche, wobei der wässrige Seitenstrom in Schritt a) mindestens 3 Gew.-%, bevorzugt mindestens 5 Gew.- %, noch bevorzugter mindestens 10 Gew.-%, selbst noch bevorzugter mindestens 20 Gew.-% und am bevorzugtesten mindestens 25 Gew.-% eines Metallcarboxylats umfasst.

6. Verfahren nach einem der vorhergehenden Ansprüche, wobei die Protonierung des Carboxylats in Richtung auf Carbonsäure in Schritt b) durch Ansäuerung des wässrigen Seitenstroms ausgeführt wird.

7. Verfahren nach einem der Ansprüche 1-5, wobei die Protonierung des Carboxylats in Richtung auf Carbonsäure in Schritt b) durch elektrochemische Membrantrennung, bevorzugt bipolare Membranelektrodialyse (BPM) des wässrigen Seitenstroms ausgeführt wird.

8. Verfahren nach einem der vorhergehenden Ansprüche, wobei das Peroxid, das in dem wässrigen Seitenstrom vorliegt, durch Zugabe eines Reduktionsmittels zu dem wässrigen Seitenstrom, entweder vor oder während Schritt b), bevorzugt vor Schritt b), zerstört wird.

9. Verfahren nach Anspruch 8, wobei das Reduktionsmittel aus der Gruppe ausgewählt wird bestehend aus Natriumsulfit, Natrium(polysulfid) (Na₂Sₓ), Natriumthiosulfat und Natriummetabisulfit.

10. Verfahren nach einem der Ansprüche 1-9, wobei die Phasen in Schritt c) durch Schwerkraft getrennt werden.

11. Verfahren nach einem der Ansprüche 1-9, wobei die Phasen in Schritt c) durch Extraktion mit einem organischen Lösungsmittel, bevorzugt einem Alkan oder einer Mischung von Alkanen, am bevorzugtesten Isododecan, getrennt werden.

12. Verfahren nach einem der Ansprüche 1-9, wobei die Phasen in Schritt c) durch Extraktion mit einer Salzlösung, bevorzugt einer NaCl-Lösung von 20-30 Gew.- %, getrennt werden.

13. Verfahren nach einem der vorhergehenden Ansprüche, umfassend einen zusätzlichen Schritt e), in dem Carbonsäure von der wässrigen flüssigen Phase durch Destillation isoliert wird .

14. Verfahren nach Anspruch 13, ferner Rückführen mindestens eines Teils der Carbonsäure, die in Schritt e) isoliert worden ist, zu der biphasischen Mischung von Schritt b) umfassend.

15. Verfahren nach einem vorhergehenden Anspruch, ferner Rückführen umfassend mindestens eines Teils der Carbonsäure, die in Schritt c) oder Schritt d) isoliert worden ist, zu einem Herstellungsvorgang für organisches Peroxid unter Anwendung der Carbonsäure, die in Schritt c) oder Schritt d) isoliert worden ist, um Ester herzustellen oder Verwenden der Carbonsäure, die in Schritt c) oder Schritt d) isoliert worden ist, in Tierfutter.

## Revendications

1. Procédé d'isolement d'acide carboxylique à partir d'un flux latéral aqueux d'un procédé de production de peroxyde organique, ledit procédé comprenant les étapes consistant à :
a) fournir un flux latéral aqueux d'un procédé de production de peroxyde organique, ledit flux comprenant au moins 1 % en poids d'un carboxylate métallique, ledit carboxylate métallique étant dissous ou mélangé de manière homogène dans ledit flux,
b) protoner le carboxylate en acide carboxylique à l'intérieur du flux latéral aqueux, formant de ce fait un mélange biphasique de deux phases liquides,
c) séparer le mélange biphasique en (i) une phase liquide aqueuse comprenant de l'eau et une quantité mineure d'acide carboxylique et (ii) une phase liquide organique comprenant l'acide carboxylique et une quantité mineure d'eau,
d) éventuellement, séparer, de préférence distiller, l'acide carboxylique de ladite phase liquide organique,
dans lequel les peroxydes résiduels présents dans le flux latéral aqueux sont éliminés par (i) extraction avant ou après l'étape b) et/ou (ii) l'ajout d'un agent réducteur, de chaleur ou d'irradiation audit flux, au mélange biphasique et/ou à la phase liquide organique.

2. Procédé selon la revendication 1 dans lequel le flux latéral aqueux résulte d'un procédé de production de peroxyde de diacyle ou de peroxyester.

3. Procédé selon la revendication 1 ou 2 dans lequel l'acide carboxylique est sélectionné dans le groupe constitué par l'acide isobutyrique, l'acide n-butyrique, l'acide propionique, l'acide pivalique, l'acide néodécanoïque, l'acide néoheptanoïque, l'acide isononanoïque, l'acide 2-méthylbutyrique, l'acide cyclohexylcarboxylique, l'acide laurique, l'acide isovalérique, l'acide n-valérique, l'acide n-hexanoïque, l'acide 2-éthylhexanoïque, l'acide heptanoïque, l'acide octanoïque, l'acide nonanoïque, l'acide décanoïque et l'acide laurique.

4. Procédé selon la revendication 3 dans lequel l'acide carboxylique est sélectionné dans le groupe constitué par l'acide isobutyrique, l'acide n-butyrique, l'acide n-heptanoïque, l'acide n-octanoïque, l'acide pivalique, l'acide isononanoïque, l'acide 2-méthylbutyrique, l'acide cyclohexylcarboxylique, l'acide isovalérique et l'acide n-valérique.

5. Procédé selon l'une quelconque des revendications précédentes dans lequel le flux latéral aqueux à l'étape a) comprend au moins 3 % en poids, de préférence au moins 5 % en poids, plus préférablement au moins 10 % en poids, encore plus préférablement au moins 20 % en poids, et le plus préférablement au moins 25 % en poids d'un carboxylate métallique.

6. Procédé selon l'une quelconque des revendications précédentes dans lequel la protonation du carboxylate en acide carboxylique à l'étape b) est réalisée par acidification du flux latéral aqueux.

7. Procédé selon l'une quelconque des revendications 1 à 5 dans lequel la protonation du carboxylate en acide carboxylique à l'étape b) est réalisée par séparation membranaire électrochimique, de préférence électrodialyse à membrane bipolaire (EDMB), du flux latéral aqueux.

8. Procédé selon l'une quelconque des revendications précédentes dans lequel le peroxyde présent dans le flux latéral aqueux est détruit par l'ajout d'un agent réducteur au flux latéral aqueux, soit avant soit pendant l'étape b), de préférence avant l'étape b).

9. Procédé selon la revendication 8 dans lequel l'agent réducteur est sélectionné dans le groupe constitué par le sulfite de sodium, un (poly)sulfure de sodium (Na₂Sₓ), le thiosulfate de sodium et le métabisulfite de sodium.

10. Procédé selon l'une quelconque des revendications 1 à 9 dans lequel les phases sont séparées à l'étape c) par gravité.

11. Procédé selon l'une quelconque des revendications 1 à 9 dans lequel les phases sont séparées à l'étape c) par extraction avec un solvant organique, de préférence un alcane ou un mélange d'alcanes, le plus préférablement l'isododécane.

12. Procédé selon l'une quelconque des revendications 1 à 9 dans lequel les phases sont séparées à l'étape c) par extraction avec une solution de sel, de préférence une solution de NaCl à 20 à 30 % en poids.

13. Procédé selon l'une quelconque des revendications précédentes comprenant une étape e) supplémentaire dans laquelle l'acide carboxylique est isolé de la phase liquide aqueuse par distillation.

14. Procédé selon la revendication 13, comprenant en outre le recyclage d'au moins une partie de l'acide carboxylique isolé à l'étape e) vers le mélange biphasique de l'étape b).

15. Procédé selon l'une quelconque des revendications précédentes, comprenant en outre le recyclage d'au moins une partie de l'acide carboxylique isolé à l'étape c) ou à l'étape d) vers un procédé de production de peroxyde organique, en utilisant l'acide carboxylique isolé à l'étape c) ou à l'étape d) pour préparer des esters, ou en utilisant l'acide carboxylique isolé à l'étape c) ou à l'étape d) dans une alimentation animale.
